# FASCICULE DE BREVET EUROPEEN

(11) **EP 0 496 648 B1**
(45) Date de publication et mention de la délivrance du brevet: **10.07.1996**
(21) Numéro de dépôt: 92400082.1
(22) Date de dépôt: 13.01.1992
(51) Int. Cl.: G01N 23/06

(54) **Procédé et dispositif de mesure des niveaux de saturation de phases multiples dans un milieu poreux**
Verfahren und Vorrichtung zur Messung des Sättigungsniveaus mehrerer Phasen in einem Porösen Medium
Process and apparatus for measuring the saturation levels of multiple phases in a porous media

(30) Priorité: 24.01.1991 FR 9100800
(43) Date de publication de la demande: 29.07.1992
(73) Titulaire: TOTAL Société anonyme dite :, F-92800 Puteaux (FR)
(72) Inventeur: Barroux, Claire Camille Olga, F-92370 Chaville (FR); Maquignon, Philippe Roger, F-91640 Briis s/Forges (FR); Thiebot, Bertrand Marie Yves, F-75014 Paris (FR)
(74) Mandataire: Jolly, Jean-Pierre

(56) Documents cités:
- FR-A- 2 455 275
- SOIL SCIENCE vol. 141, no. 2, Février 1986, USA, pages 163-171; M.E. GRISMER ET AL.: 'MONITORING WATER AND SALT MOVEMENT IN SOILS AT LOW SOLUTIONS CONTENTS'
- REVUE DE L'INSTITUT FRANCAIS DU PETROLE vol. 41, no. 3, Mai 1986, PARIS FR, pages 395-404; "Recommandations pour la détermination des profils de porosité et de saturation dans les milieux poreux par absorption d'un rayonnement X"

## Description

La présente invention concerne un procédé et un dispositif de mesure des niveaux de saturation des phases multiples dans un milieu poreux par absorptiométrie de rayons gamma à partir de deux sources radio-actives utilisées en alternance. Elle concerne plus particulièrement la mesure, lors d'un écoulement en milieu poreux, des niveaux de saturation moyens de trois phases, eau, huile et gaz existant en divers points et instants de ce milieu.

L'invention concerne en particulier le domaine des hydrocarbures et gisements pétroliers.

L'absorptiométrie gamma à deux sources radioactives utilisées en alternance pour mesurer les teneurs en constituants d'un milieu à divers endroits et instants est connue. L'article SOIL SCIENCE Vol. 141, N° 2 de Février 1986 décrit à ce sujet un système d'absorptiométrie à deux sources radioactives pour la mesure des teneurs en eau et en sels d'un sol. Ce système utilise deux sources radioactives logées dans des barillets coaxiaux et dont la rotation de l'un par rapport à l'autre permet par des trous de collimation adéquats de faire collimater les rayons de ces sources en alternance sur une même fenêtre de collimation de façon qu'un volume donné du milieu analysé en regard de ladite fenêtre puisse être scruté individuellement (ou même simultanément) et à faible intervalle de temps par chacune de ces sources. L'une de ces sources est logée en position centrale dans le barillet le plus interne tandis que l'autre est positionnée sur le barillet externe et dans une position proche de la périphérie. Ces sources n'étant pas logées à la même profondeur en regard de la fenêtre de collimation du rayonnement, il en résulte que les volumes scrutés par chacune des deux sources ne sont pas exactement les mêmes et par suite les résultats de mesure déduits des équations de Beer-Lambert, qui supposent des volumes scrutés parfaitement identiques, ne peuvent pas être de la meilleure précision.

Par ailleurs, un but de l'invention est de proposer un système automatisé permettant la mesure ponctuellement sur le milieu analysé et avec déplacement le long de ce milieu, qui est peut être un échantillon de roche par exemple.

A cet effet, l'invention propose un procédé de mesure des saturations moyennes d'une pluralité de phases en différents emplacements d'un échantillon d'un milieu poreux, du type consistant à collimater successivement ou alternativement le rayonnement de deux sources de rayonnement gamma d'énergies différentes sur chacun desdits emplacements du milieu poreux, à mesurer pour chacune desdites sources et en chacun desdits emplacements l'intensité du rayonnement qu'elles émettent et l'intensité du rayonnement ayant traversé l'échantillon du milieu poreux et à calculer à partir de ces intensités la saturation moyenne des différentes phases en chacun de ces emplacements, ce procédé étant caractérisé en ce que l'on déplace par rapport à l'échantillon un équipage mobile comportant un premier et un second chariots rigidement solidaires l'un de l'autre, de manière à amener chacun des chariots en regard des emplacements de l'échantillon respectivement de part et d'autre de cet échantillon, le premier chariot portant les deux sources de rayonnement gamma et un système de collimation de leur rayonnement, tandis que le second chariot porte, en regard du système de collimation, un appareil de mesure de l'intensité ayant traversé l'échantillon, la source d'énergie la plus faible étant fixe en position sur le premier chariot et disposée suivant l'axe de collimation dudit système, tandis que la seconde source est montée mobile sur le premier chariot, des moyens étant prévus pour amener cette seconde source suivant l'axe de collimation dudit système, à proximité de la première, ou à l'écart de cet axe.

L'invention concerne également un dispositif pour la mise en oeuvre du procédé susmentionné.

Ce dispositif est donc un dispositif de mesure des saturations moyennes d'une pluralité de phases en différents emplacements d'un échantillon d'un milieu poreux, ce dispositif comprenant deux sources de rayon gamma d'énergies différentes, des moyens pour collimater le rayonnement de ces sources successivement ou alternativement sur lesdits emplacements du milieu poreux et des moyens de mesure de l'intensité du rayonnement émise par chacune desdites sources et l'intensité du rayonnement ayant traversé l'échantillon en chacun desdits emplacements, ce dispositif étant caractérisé en ce qu'il comprend un équipage mobile et des moyens pour déplacer cet équipage mobile par rapport audit échantillon, l'équipage mobile comportant un premier et un second chariots rigidement solidaires l'un de l'autre et disposés de façon telle qu'ils puissent être placés de part et d'autre de l'échantillon et les moyens de déplacement de cet équipage étant aptes à amener chacun des chariots en regard des emplacements de mesure de l'échantillon, la source d'énergie la plus faible étant fixe en position sur le premier chariot et disposée suivant l'axe de collimation dudit système, tandis que la seconde source est montée mobile sur le premier chariot, des moyens étant prévus pour amener cette seconde source suivant l'axe de collimation dudit système, à proximité de la première, ou à l'écart de cet axe.

Ce dispositif comporte un boîtier blindé mobile, logeant lesdites deux sources radioactives utilisées en alternance, et dont le rayonnement émis est collimaté par un élément ou bloc de collimation obturable, la source de plus faible énergie étant fixée à demeure, sensiblement en position centrale dans le boîtier, et dans l'axe de collimation du dispositif.

La première source ou source de plus faible énergie est avantageusement une source d'américium et la seconde une source de césium.

Il résulte donc de cette disposition que le rayonnement de la source fixe d'américium peut scruter individuellement le milieu à analyser, la source de césium étant occultée hors de l'axe de collimation et que le rayonnement de cette seconde source peut être exploité individuellement lorsqu'elle est amenée dans l'axe de collimation. Ces sources étant situées sensiblement à la même profondeur en service au sein du boîtier, l'angle solide de collimation de rayonnement de chacune des sources, délimité par les rayons extrêmes émis, est pratiquement le même de sorte que les volumes scrutés par ces rayonnements au sein du milieu, sur une distance déterminée, sont également pratiquement identiques.

Cette disposition est importante comme déjà indiqué ci-dessus pour la précision des résultats déduits ultérieurement des mesures.

Le bloc de collimation obturable peut être un barillet blindé monté rotatif au sein du boîtier porte-sources entre les sources et le milieu à analyser, et dont le trou de collimation est dirigé dans l'axe de collimation en service.

La seconde source peut également être fixée sur un barillet monté rotatif au sein du boîtier et qu'on meut à un angle de rotation déterminé pour positionner ladite source en service à proximité de la première et dans l'axe de collimation. Dans les autres positions, cette source est occultée

Selon une disposition constructive avantageuse, lesdits deux barillets, respectivement obturateur-collimateur et porte-sources sont disposés au sein du boîtier avec leur axe de rotation parallèles à l'axe de collimation et avec symétrie sensiblement par rapport à la première source, la seconde source étant fixée sur la face la plus interne de son barillet support.

Ces barillets sont mis en rotation à convenance par des moteurs électriques, de même que le déplacement des chariots de l'équipage mobile, sous la dépendance d'une unité de commande adéquate.

L'invention est illustrée ci-après à l'aide d'un exemple de réalisation et en référence aux dessins annexés sur lesquels :
la figure 1 est une vue schématique réduite de l'ensemble expérimental dans lequel s'insère le dispositif d'absorptiométrie selon l'invention,
la figure 2 est une vue schématique du dispositif d'absorptiométrie seul et
la figure 3 est une vue agrandie du boîtier-porte-sources de ce dispositif.

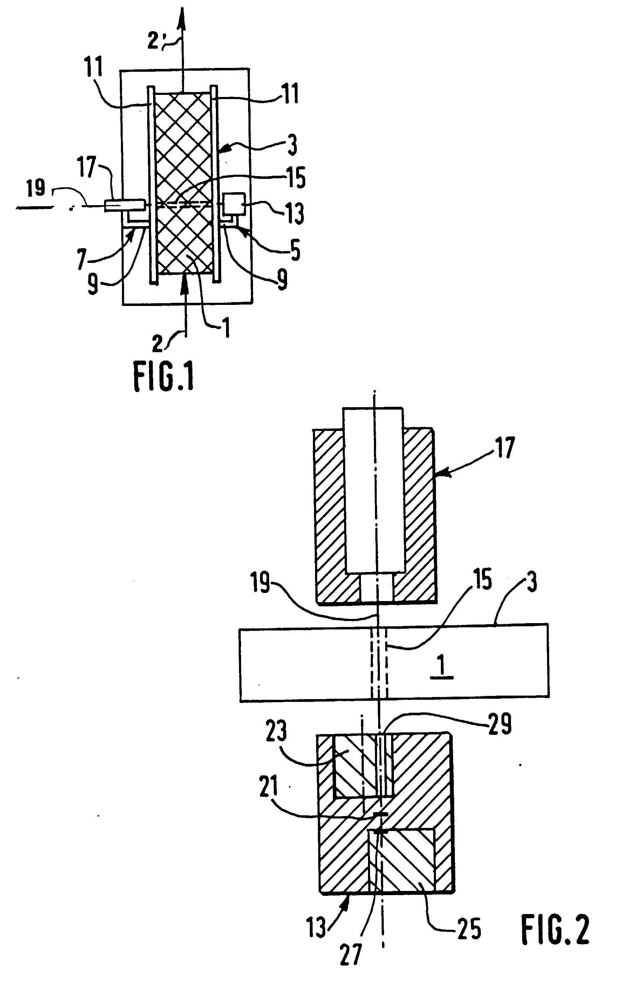

Comme représenté sur la figure 1, le dispositif selon l'invention s'insère dans un ensemble expérimental de mesure comprenant un milieu poreux 1, contenu dans une cellule de contionnement, et dans lequel s'écoule par les conduites 2 et 2′ un fluide à pression et température déterminées. Ce fluide se répartit en des phases multiples, eau, huile et gaz dont les niveaux de saturation moyens sont à analyser. Ce milieu est par exemple un échantillon de roche dans lequel s'écoule un hydrocarbure, permettant ainsi de simuler les conditions existant dans les gisements pétroliers. De part et d'autre de ce milieu sont disposés respectivement un chariot porte-sources 5 et un chariot détecteur de rayonnement 7. Ces chariots sont montés solidaires l'un de l'autre, par exemple par une même jambe inférieure 9, mobile verticalement. Ils se déplacent ainsi verticalement, parallèlement le long du milieu, conditionné dans l'exemple par des parois 11 transparentes au rayonnement, qui sont disposées verticalement.

Le chariot porte-sources comporte un boîtier blindé 13 qui loge les sources radio-actives de rayonnement. Par ce boîtier est collimaté un rayonnement à travers le milieu dans un volume sensiblement linéaire 15 de celui-ci. Ce rayonnement, absorbé partiellement par le milieu, est reçu par un élément détecteur 17 centré sur l'axe de collimation 19 et porté par le second chariot. Le dispositif est mobile sur la hauteur de l'échantillon, se déplaçant pour permettre la mesure en divers points de cet échantillon, mesures qui sont effectuées naturellement à l'arrêt.

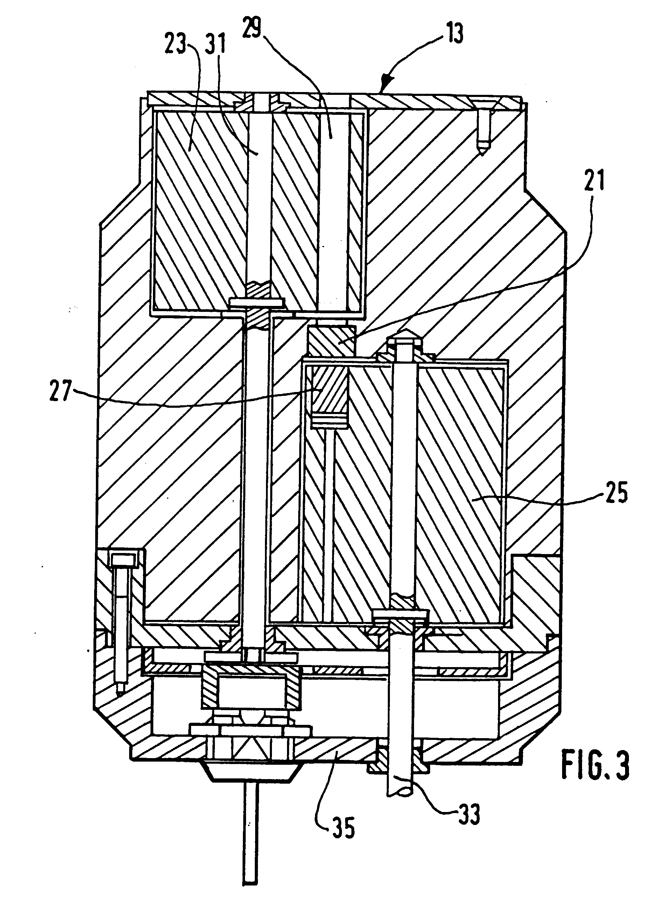

Le détail du dispositif d'absorptiométrie proprement dit est explicité à la figure 2. Seuls sont représentés la cellule de conditionnement 3 contenant le milieu poreux analysé 1, le boîtier porte-sources 13 et le bloc détecteur 17. Le boîtier porte-sources 13, monté sur son chariot support, comporte une pastille d'américium 21 en position fixe centrale (première source radio-active), un barillet obturateur-collimateur supérieur 23 et un barillet inférieur 25 portant sur sa face supérieure une pastille de césium 27 (seconde source radioactive). Ce boîtier est en matériau blindé opaque au rayonnement, de forme cylindrique et son diamètre est sensiblement équivalent à sa hauteur. L'axe de collimation du rayonnement 19 est aussi l'axe de révolution du boîtier qui passe par la source radioactive américium 21. Les barillets obturateur-collimateur 23 et support de la source de césium 25 ont leur axe parallèle à l'axe de collimation. Le barillet obturateur-collimateur 23 comporte un trou longitudinal à faible diamètre 29, parallèle à l'axe de collimation 19, formant fonction de fenêtre de collimation à un angle de rotation donné du barillet. A toute autre position angulaire du barillet sur son axe, la matière blindée opaque au rayonnement constitutive du barillet, occulte le rayonnement radioactif. Le second barillet 25 est disposé sensiblement symétrique du premier par rapport à la source d'américium. La source de césium à haute énergie qu'il porte est disposée sur sa face supérieure à un rayon tel qu'à un angle donné de rotation du barillet, elle se trouve également dans l'axe de collimation et à faible distance de la source d'américium (position de service). A toute autre position angulaire du barillet, son rayonnement radioactif est occulté, ce qui l'empêche d'interférer avec celui de la source d'américium de basse énergie qui peut donc être exploité seul sans l'effet compton parasite bien connu engendré par la source à haute énergie. Ces barillets sont mis en rotation (figure 3) par leur axe support respectif 31,33, monté sur la carcasse 35 du boîtier. L'actionnement en rotation est réalisé par des moteurs électriques adéquats (non représentés).

Le bloc détecteur 17 est un photomultiplicateur classique du rayonnement radioactif. Ce bloc comme précisé antérieurement, est centré sur l'axe de collimation du boîtier porte-sources, se déplaçant solidairement avec ce dernier le long de l'échantillon, à l'opposé de celui-ci.

Le fonctionnement du dispositif est le suivant.

Pendant la mesure, on déplace le long du milieu poreux le boîtier porte sources 13 et le bloc détecteur 17 à l'aide de l'équipage mobile à chariots supports correspondants. On arrête le dispositif à chaque point de mesure. Une première mesure s'effectue avec la source d'américium 21 seule, la source de césium 27 étant décalée de l'axe de collimation. Pour cela, le bloc collimateur-obturateur 23 est pivoté de manière à découvrir la fenêtre de collimation 29 (trou collimateur sur l'axe de collimation) et le barillet inférieur est tourné autour de son axe de façon à occulter le rayonnement du césium. Une deuxième mesure s'effectue en exploitant le rayonnement de la mesure de la source de césium, dont le barillet-support 25 est tourné de façon à l'amener sur l'axe de collimation 19. Son rayonnement est alors collimaté sur le milieu et analysé.

Les mouvements de l'ensemble, équipage mobile et barillets sont asservis à une unité de commande du type micro-ordinateur.

L'exemple de réalisation ci-dessus décrit l'utilisation de deux sources radioactives d'énergie différentes mais il est évident que des variantes de réalisation à plusieurs sources radioactives, une fixe et d'autres réparties sur le barillet-support inférieur, peuvent entrer dans le cadre de l'invention revendiquée ci-après.

## Revendications

1. Procédé de mesure des saturations moyennes d'une pluralité de phases en différents emplacements d'un échantillon d'un milieu poreux (1), du type consistant à collimater successivement ou alternativement le rayonnement de deux sources (21,27) de rayonnement gamma d'énergies différentes sur chacun desdits emplacements du milieu poreux, à mesurer pour chacune desdites sources et en chacun desdits emplacements l'intensité du rayonnement qu'elles émettent et l'intensité du rayonnement ayant traversé l'échantillon du milieu poreux et à calculer à partir de ces intensités la saturation moyenne des différentes phases en chacun de ces emplacements, ce procédé étant caractérisé en ce que l'on déplace par rapport à l'échantillon un équipage mobile (5,7,9), comportant un premier et un second chariots rigidement solidaires l'un de l'autre, de manière à amener chacun des chariots en regard des emplacements de l'échantillon respectivement de part et d'autre de cet échantillon, le premier chariot (5) portant les deux sources de rayonnement gamma et un système de collimation de leur rayonnement, tandis que le second chariot (7) porte, en regard du système de collimation, un appareil de mesure de l'intensité ayant traversé l'échantillon, la source d'énergie la plus faible (21) étant fixe en position sur le premier chariot et disposée suivant l'axe de collimation (19) dudit système, tandis que la seconde source (27) est montée mobile sur le premier chariot, des moyens étant prévus pour amener cette seconde source suivant l'axe de collimation (19) dudit système, à proximité de la première, ou à l'écart de cet axe.

2. Dispositif de mesure des saturations moyennes d'une pluralité de phases en différents emplacements d'un échantillon d'un milieu poreux (1), ce dispositif comprenant deux sources (21,27)de rayon gamma d'énergies différentes, des moyens pour collimater le rayonnement de ces sources successivement ou alternativement sur lesdits emplacements du milieu poreux et des moyens de mesure de l'intensité du rayonnement émise par chacune desdites sources et l'intensité du rayonnement ayant traversé l'échantillon en chacun desdits emplacements, ce dispositif étant caractérisé en ce qu'il comprend un équipage mobile (5,7,9) et des moyens pour déplacer cet équipage mobile par rapport audit échantillon, l'équipage mobile comportant un premier et un second chariots rigidement solidaires l'un de l'autre et disposés de façon telle qu'ils puissent être placés de part et d'autre de l'échantillon et les moyens de déplacement de cet équipage étant aptes à amener chacun des chariots en regard des emplacements de mesure de l'échantillon, la source d'énergie la plus faible (21) étant fixe en position sur le premier chariot (5) et disposée suivant l'axe de collimation (19) dudit système, tandis que la seconde source (27) est montée mobile sur le premier chariot, des moyens étant prévus pour amener cette seconde source suivant l'axe de collimation (19) dudit système, à proximité de la première, ou à l'écart de cet axe.

3. Dispositif selon la revendication 2, caractérisé en ce qu'il comporte un boîtier blindé mobile (13), logeant lesdites deux sources radioactives (21,27) utilisées en alternance, et dont le rayonnement émis est collimaté par un élément ou bloc de collimation obturable (23), la source de plus faible énergie (21) étant fixée à demeure sensiblement en position centrale dans le boîtier et dans l'axe de collimation (19) du dispositif.

4. Dispositif selon l'une des revendications 2,3, caractérisé en ce que la première source radioactive (21) est constitué par une pastille d'américium et la seconde (27) par une pastille de césium.

5. Dispositif selon l'une des revendications 2 à 4, caractérisé en ce que le bloc de collimation obturable est un barillet (23) en matériau blindé, opaque au rayonnement, monté rotatif au sein du boîtier porte-sources (13), entre les sources radioactives et le milieu à analyser, et dont le trou de collimation (29) est dirigé dans l'axe de collimation (19) en service.

6. Dispositif selon l'une des revendications 2 à 5, caractérisé en ce que la seconde source (27) est fixée sur un barillet (25) monté rotatif au sein du boîtier (13) et qu'on meut à un angle de rotation déterminé pour positionner ladite source en service à proximité de la première et dans l'axe de collimation (19).

7. Dispositif selon l'une des revendications 2-6, caractérisé en ce que lesdits deux barillets, respectivement obturateur-collimateur (23) et porte sources inférieur (25) sont disposés au sein du boîtier avec leur axe de rotation parallèles à l'axe de collimation (19) et avec symétrie sensiblement par rapport à la première source (21), la seconde source (27) étant fixée sur la face la plus interne de son barillet support.

8. Dispositif selon l'une des revendications 2,7, caractérisé en ce que lesdits barillets (23,25) sont mis en rotation à convenance par des moteurs électriques, de même que le déplacement des chariots (5,7) de l'équipage mobile, sous la dépendance d'une unité de commande adéquate.

9. Dispositif selon l'une des revendications 2-8, caractérisé en ce que le barillet inférieur (25) porte plusieurs sources radioactives.

10. Dispositif selon l'une des revendications 2-9, caractérisé en ce que le boîtier (13) a la forme d'un cylindre ayant pour axe de révolution l'axe de collimation (19) et dont le diamètre est sensiblement équivalent à la hauteur.

## Patentansprüche

1. Verfahren zur Messung des Sättigungsniveaus mehrerer Phasen an unterschiedlichen Stellen einer Probe eines porösen Mediums (1), bei dem die Strahlung von zwei Gammastrahlenquellen (21, 27) von unterschiedlicher Energie nacheinander oder abwechselnd auf jede der Stellen des porösen Mediums ausgerichtet wird, für jede der genannten Quellen und an jeder der genannten Stellen die von ihnen ausgesendete Strahlungsintensität bzw. die die Probe des porösen Mediums durchquerte Strahlungsintensität gemessen und aus diesen Intensitäten die mittlere Sättigung der unterschiedlichen Phasen an jeder der Stellen berechnet wird,
**dadurch gekennzeichnet,**
daß man eine bezüglich der Probe bewegliche Bauteilgruppe (5, 7, 9), die einen ersten und einen zweiten, miteinander in fester Verbindung stehenden Schlitten umfaßt, derart verfährt, daß jeder der beiderseits der Probe befindlichen Schlitten jeweils an die Stellen der Probe herangeführt wird,
- der erste Schlitten (5) die beiden Gammastrahlenquellen sowie ein Kollimationssystem für ihre Strahlung und der zweite Schlitten (7) gegenüber dem Kollimationssystem ein Meßgerät für die Intensität der die Probe durchquerten Strahlung trägt,
- die Strahlungsquelle (21) mit der kleinsten Energie in Einsatzstellung fest auf dem ersten Schlitten und der Achse (19) des Kollimationssystems folgend und die zweite Strahlungsquelle (27) auf dem ersten Schlitten beweglich angeordnet ist,
- Mittel vorgesehen sind, um die nahe der ersten Strahlungsquelle oder mit Abstand von der Achse (19) des Kollimationssystems angeordnete zweite Strahlungsquelle in Ausrichtung zur Kollimationsachse zu bringen.

2. Vorrichtung zur Messung des Sättigungsniveaus mehrerer Phasen an unterschiedlichen Stellen einer Probe eines porösen Mediums (1), die zwei Gammastrahlenquellen (21, 27) von unterschiedlicher Energie, Mittel zum aufeinanderfolgenden oder abwechselnden Ausrichten der Strahlung dieser Strahlungsquellen auf die Stellen des porösen Mediums sowie Einrichtungen zur Intensitätsmessung der von jeder der Strahlungsquellen ausgehenden sowie derjenigen Strahlung enthält, welche die Probe an jeder der genannten Stellen durchlaufen hat,
**dadurch gekennzeichnet,**
daß sie eine bewegliche Bauteilgruppe (5, 7, 9) sowie Einrichtungen zur Verstellung der beweglichen Bauteilgruppe bezüglich der Probe aufweist,
- die bewegliche Bauteilgruppe einen ersten und einen zweiten Schlitten aufweist, die in starrer Verbindung zueinander so angeordnet sind, daß sie zu beiden Seiten der Probe eingestellt werden können, wobei Verstelleinrichtungen für die Bauteilgruppe in der Lage sind, jeden der Schlitten an die Meßstellen der Probe heranzuführen,
- die Strahlungsquelle mit der geringsten Energie (21) auf dem ersten Schlitten (5) in Einsatzstellung befestigt und der Kollimationsachse (19) des Systems folgend angeordnet ist, während die zweite Strahlungsquelle (27) auf dem ersten Schlitten beweglich montiert ist,
- Mittel vorgesehen sind, um die nahe der ersten Strahlungsquelle oder mit Abstand von der Achse (19) des Kollimationssystems angeordnete zweite Strahlungsquelle in Ausrichtung zur Kollimationsachse zu bringen.

3. Vorrichtung nach Anspruch 2, dadurch gekennzeichnet, daß sie ein bewegliches gepanzertes Gehäuse (13) umfaßt, in dem die beiden abwechselnd benutzten radioaktiven Strahlungsquellen (21, 27) untergebracht sind, deren emittierte Strahlung mittels eines absperrbaren Kollimationsgliedes oder -blocks (23) gerichtet wird, wobei die Strahlungsquelle mit der geringsten Energie (21) so befestigt ist, daß sie sich im wesentlichen an zentraler Stelle im Gehäuse und in der Kollimationsachse (9) der Vorrichtung befindet.

4. Vorrichtung nach Anspruch 2 oder 3, dadurch gekennzeichnet, daß die erste radioaktive Strahlungsquelle (21) aus einer Amerizium-Tablette und die zweite Strahlungsquelle (27) aus einer Cäsium-Tablette besteht.

5. Vorrichtung nach einem der Ansprüche 2 bis 4, dadurch gekennzeichnet, daß der absperrbare Kollimationsblock ein für Strahlung undurchlässiger, aus panzerartig wirkendem Material bestehender Zylinderkörper (23) ist, der innerhalb des die Strahlungsquellen enthaltenden Gehäuses (13) zwischen diesen und dem zu untersuchenden Medium drehbar gelagert ist und dessen Kollimationsbohrung (29) in Betrieb auf die Kollimationsachse (19) eingestellt ist.

6. Vorrichtung nach einem der Ansprüche 2 bis 5, dadurch gekennzeichnet, daß die zweite Strahlungsquelle (27) auf einem innerhalb des Gehäuses (13) drehbar gelagerten Zylinderkörper (25) befestigt ist, der um einen bestimmten Drehwinkel bewegbar ist, um im Betrieb die genannte Strahlenquelle in die Nähe der ersten Strahlenquelle und auf die Kollimationsachse (19) einzustellen.

7. Vorrichtung nach einem der Ansprüche 2 bis 6, dadurch gekennzeichnet, daß die beiden Zylinderkörper bzw. das Absperr-Kollimatorglied (23) und das untere Strahlungsquellen-Trägerglied (25) mit ihren zur Kollimationsachse (19) parallelen Drehachsen sowie zur ersten Strahlungsquelle (21) im wesentlichen symmetrisch innerhalb des Gehäuses angeordnet sind, und daß die zweite Strahlungsquelle (27) auf der im Gehäuse am weitesten innen befindlichen Oberfläche des ihn tragenden Zylinderkörpers befestigt ist.

8. Vorrichtung nach einem der Ansprüche 2, 7, dadurch gekennzeichnet, daß die Drehverstellung der Zylinderkörper (23, 25) wie auch die Verstellbewegung der Schlitten (5, 7) der beweglichen Bauteilgruppe beliebig durch Elektromotoren in Abhängigkeit von einer geeigneten Steuereinheit erfolgt.

9. Vorrichtung nach einem der Ansprüche 2 bis 8, dadurch gekennzeichnet, daß der untere Zylinderkörper (25) mehrere radioaktive Strahlungsquellen trägt.

10. Vorrichtung nach einem der Ansprüche 2 bis 9, dadurch gekennzeichnet, daß die Mittel-/Drehachse des zylinderförmig ausgebildeten Gehäuses (13) mit der Kollimationsachse (19) zusammenfällt und daß der Durchmesser des Gehäuses im wesentlichen der Gehäusehöhe gleicht.

## Claims

1. Method for measuring the mean saturations of a plurality of phases at different locations in a sample of a porous medium (1), of the type consisting of collimating, in succession or in alternation, the radiation from two sources (21, 27) of gamma radiation of different energies at each of the said locations in the porous medium, measuring, for each of the said sources and at each of the said locations, the intensity of the radiation which they are emitting and the intensity of the radiation which has passed through the sample of porous medium, and calculating, from these intensities, the mean saturation of the difference phases at each of these locations, this method being characterised in that a movable apparatus (5, 7, 9), including first and second carriages rigidly fixed to each other, is moved with respect to the sample so as to bring each of the carriages opposite the locations in the sample respectively on each side of this sample, the first carriage (5) carrying the two gamma radiation sources and a system for collimating their radiation, whilst the second carriage (7) carries, opposite the collimation system, equipment for measuring the intensity which has passed through the sample, the weakest energy source (21) being fixed in position on the first carriage and disposed on the collimation axis (19) of the said system, whilst the second source (27) is movably mounted on the first carriage, means being provided for bringing this second source on the collimation axis (19) of the said system, close to the first, or at a distance from this axis.

2. Device for measuring the mean saturations of a plurality of phases at different locations in a sample of a porous medium (1), this device comprising two sources (21, 27) of gamma radiation of different energies, means for collimating the radiation of these sources, in succession or in alternation, at the said locations in the porous medium, and means for measuring the intensity of the radiation emitted by each of the said sources and the intensity of the radiation which has passed through the sample at each of the said locations, this device being characterised in that it comprises a movable apparatus (5, 7, 9) and means for moving this movable apparatus with respect to the said sample, the movable apparatus including first and second carriages rigidly fixed to each other and disposed so that they can be positioned on each side of the sample and the means of moving this apparatus being suitable for bringing each of the carriages opposite the sample measurement locations, the weakest energy source (21) being fixed in position on the first carriage (5) and disposed on the collimation axis (19) of the said system, whilst the second source (27) is movably mounted on the first carriage, means being provided for bringing this second source on the collimation axis (19) of the said system, close to the first, or at a distance from this axis.

3. Device according to Claim 2, characterised in that it includes a movable shielded case (13), housing the said two radioactive sources (21, 27) used in alternation, and whose emitted radiation is collimated by a collimation element or block (23) which can be shut, the weakest energy source (21) being permanently fixed substantially in a central position in the case and on the collimation axis (19) of the device.

4. Device according to one of Claims 2, 3, characterised in that the first radioactive source (21) consists of a pellet of americium and the second (27) a pellet of caesium.

5. Device according to one of Claims 2 to 4, characterised in that the collimation block which can be shut is a pot (23) made of shielded material, opaque to radiation,
rotatably mounted within the source-holder case (13), between the radioactive sources and the medium to be analysed, and whose collimation aperture (29) is directed along the collimation axis (19) in service.

6. Device according to one of Claims 2 to 5, characterised in that the second source (27) is fixed to a pot (25) rotatably mounted within the case (13) and which is moved at a given angle of rotation in order to position the said source in service close to the first and on the collimation axis (19).

7. Device according to one of Claims 2-6, characterised in that the said two pots, respectively the shutter/collimator pot (23) and the lower source-holder pot (25), are disposed within the case with their axis of rotation parallel to the collimation axis (19) and with symmetry substantially with respect to the first source (21), the second source (27) being fixed to the innermost face of its holder pot.

8. Device according to one of Claims 2, 7, characterised in that the said pots (23, 25) are rotated as required by electric motors, just like the movement of the carriages (5, 7) of the movable apparatus, under the control of a suitable control unit.

9. Device according to one of Claims 2-8, characterised in that the lower pot (25) holds several radioactive sources.

10. Device according to one of Claims 2-9, characterised in that the case (13) has the shape of a cylinder having the collimation axis (19) as its axis of rotation and whose diameter is substantially equivalent to its height.
